# EUROPEAN PATENT APPLICATION

(11) **EP 3 171 170 A1**
(43) Date of publication of application: **24.05.2017**
(21) Application number: 15194955.9
(22) Date of filing: 17.11.2015
(51) Int. Cl.: G01N 33/52, G01N 33/543

(54) **DEVICE FOR USE IN ELISPOT**

(71) Applicant: Lionex GmbH, 38126 Braunschweig (DE)
(72) Inventor: Singh, Mahavir, 38124 Braunschweig (DE); Truthmann, Katja, 38126 Braunschweig (DE)

(57) **Abstract**

In a first aspect, the present invention relates to the use of a device, like a microtiter plate having multiple predetermined reaction areas, like multiple wells, wherein the device with the multiple predetermined reaction areas, like the wells of the microtiter plate, do not contain any membrane or other structure present in or on the predetermined reaction areas, e.g. fitted into the well, for use in an enzyme-linked immunospot (ELISPOT) assay. Surprisingly, the inventors identified that the ELISPOT assay can be performed using simple devices the the multiple predetermined reaction areas, like the microtiter plates without the need of any kind of membrane or other structure present in or on the reaction areas of the deivce, like fitted into the well, for increasing surface area etc. In a further aspect, the present invention relates to an ELISPOT assay based method for determining compounds secreted from culture cells whereby the culturing of the culture cells is conducted in or on the device, like in the microtiter plates according to the present invention. Furthermore, a test kit for use in an ELISPOT assay comprising the devices, like the microtiter plates according to the present invention are provided.

## Description

In a first aspect the present invention relates to the use of a device, like a microtiter plate, having multiple predetermined reaction areas, like multiple wells, wherein the device with the multiple predetermined reaction areas, like the wells of the microtiter plate, do not contain any membrane or other structure present in or on the predetermined reaction areas, e.g. fitted into the well, for use in an enzyme-linked immunospot (ELISPOT) assay. Surprisingly, the inventors identified that the ELISPOT assay can be performed using simple devices with the multiple predetermined reaction areas, like the microtiter plates, without the need of any kind of membrane or other structure present in or on the reaction areas of the device, like fitted into the well, for increasing surface area etc. In a further aspect, the present invention relates to an ELISPOT assay based method for determining compounds secreted from culture cells whereby the culturing of the culture cells is conducted in or on the device, like in the microtiter plates, according to the present invention. Furthermore, a test kit for use in an ELISPOT assay comprising the devices, like the microtiter plates, according to the present invention are provided.

### Prior art

The enzyme-linked immunospot (ELISPOT) assay is a very sensitive immunoassay, which allows the detection of secreted analyte at the single cell level. That is, the detection levels being as low as one cell in 100.000 cells, the ELISPOT is one of the most sensitive cellular assays available. Generally, the sensitivity compared to a conventional ELISA is about 20 to 200 times more sensitive when analysing secreted analytes of cytokine or other secreted factors.

That is, the ELISPOT has similar sensitivity to RT-PCR analysis but detects secreted protein instead of mRNA. This is of particular advantage since mRNA does not automatically result in the translated peptide or protein but detection of secreted protein is a more reliable measure. For cytokine analysis, one of the main field of the application of ELISPOT, the analysis on the protein level is more advantageous since many cytokines are translationally regulated. Moreover, while mRNA analysis requires extensive preparation of the sample to make the mRNA available, e.g. for PCR analysis, the detection of secreted analyte including cytokines is more accurate and is linked with less errors based on laborious work resulting in hand made errors.

ELISPOT analysis is also less impaired by binding proteins and protease activity since the analyte is bound to the capture antibody immediately after secretion. It has been proven that ELISPOT represents a powerful tool useful when studying small populations of cells such as those regularly found in specific immune responses in view of its high sensitivity.

Hence, ELISPOT assays has been widely applied to investigate and monitor cellular immune responses in humans and other animals and has found clinical applications in the diagnosis of e.g. tuberculosis, in the monitoring of graft tolerance or rejection in transplant patients. In addition, ELISPOT is applied in clinical applications in infections, cancer, allergies and autoimmune diseases. It has become a standard tool in development and monitoring of new vaccines and vaccine candidate. For example, the ELISPOT assays allows to identify subgroups of immune cells including T-helper cells, T-regulatory cells etc. In addition, the ELISPOT assay is suitable for performing individual tests but also large scale trials.

The typical application of ELISPOT is the identification and enumeration of cytokines producing cells at a single cell level.

The ELISPOT assay allows visualization of the secretory product of individually activated or responding cells whereby each spot that develops in the assay represents a single reactive cell. Consequently, the ELISPOT assay represents an assay providing both qualitative and quantitative information on the specific analyte secreted by the cells accordingly. Due to the exquisite sensitivity of ELISPOT assays, analysis of the frequency of rare antigen specific cells within a test population, which had been impossible to perform prior to its development, have now become relatively simple.

An important advance in recent years has been the development of in vitro T cell-based cytokine release assays (e.g. IGRAs). Besides the high specificity, other potential advantages of IGRA's include logistical convenience, avoidance of subjective measurement (such as skin induration), need for fewer patient visits, and the ability to perform serial testing without inducing the boosting phenomenon [Chiappini E, et al. (2012), PLoS ONE 7(9): e46041. doi:10.1371/jour-nal.pone.0046041; Madhukar Pai and Dick Menzies, Dewan et al. (2007), EDITORIAL COMMENTARY, CID 2007:44 (1 January), 74, page 69-73]. Elispot is the only method for the quantitation of actual secretory activity of individual cells. Intracellular cytokine staining (ICS), flow cytomtery or mRNA measurement do not provide comparable and detailed information as ELISPOT [Rininsland, et al., (2000), J Immunol Methods 240, 143-155; Kleen, T.O., et al., (2005). AIDS 18, 383-39; Kuerten, S., et al., (2007) AIDS Research and Human Retrovirus 24, 62-71; S., Asaad, R.J., et al., (2008) AIDS Res Hum Retrovirus 24, 1-9]. In all cases, the standard ELISPOT micro titer plate contains PVDF membranes.

That is, today the ELISPOT assay is based on an assay performed in a microtiter plate having a membrane surface, typically, a 96 well PVDF membrane microtiter plate. The membrane present in the microtiter plate are coated with capture antibody that binds a specific epitope of the secreted analyte being assayed.

The cells are incubated and stimulated, e.g. PBMC are seeded into the wells of the plate along with the antigen and form a monolayer on the membrane surface of the wells. Due to the activation of the cells, the cells secrete compounds including the analyte to be analysed, e.g. the cytokine, into the environment. This analyte is captured directly on the membrane surface by the immobilized antibody, thus, capturing the analyte directly on the surrounding of the secreting cell. Hence, it is possible to allocate secretion to the secreting cell before diffusion of said analyte in the culture supernatant. Furthermore, by direct capturing of the analyte, the possibility of degradation due to proteases present in the supernatant or released by the cells or the unrelated allocation of the secretion to other cells due to binding to corresponding receptors on that cells is reduced.

Detection of the secreted and immobilized analyte is conducted usually by immunospot assay. Thus, it is possible to have a secretory footprint of the activated cell.

That is, the procedure of the ELISPOT assay is typically as follows: A monoclonal or polyclonal capture antibody is coated under sterile conditions onto a PVDF (polyvinylidene fluoride) backed microplate. After coating, the plates are blocked usually using a serum protein being unreactive with any of the antibodies in the assay. Thereafter, the cells of interest are introduced into the wells of the microplates and incubated under predetermined conditions in a humidified incubator for the desired period of time.

The secreted cell product of interest, like the cytokine secreted by the cultured cells, is captured locally by the coated antibody on the high surface area of PVDF membrane. The wells are washed to remove supernatant cells or debris and, thereafter, a detection antibody being directed to the cell product of interest, typically labelled with a label like biotin, is incubated and, thereafter, visualizing is conducted using florescent dyes or enzyme products including horseradish peroxidase or alkaline phosphatase. The coloured end product, namely, the spot, can be analysed in order to analyse spot numbered size. The method can be conducted for qualitative or quantitative analysis.

The ELISPOT is used for *in vitro* tests based on detection of cell-mediated immune reaction *Mycobacterium tuberculosis* (MTB) since 2011. Therein, *Mycobacterium tuberculosis* specific antigens, ESAT-6 and CFP-10 peptides, are used to stimulate MTB-sensitized T-cells for the production of interferon-gamma. Thus, it is possible to determine the presence of MTB-specific cells, allowing the diagnosis of tuberculosis.

However, the ELISPOT assays described so far require the use of cost expensive membrane containing microtiter plates. E.g., the costs of a 96-well PVDF-membrane microtiter plate to be used in ELISPOT assay in 2015 amounts to about 70 euros.

Thus, routine use of the ELISPOT system is hindered in view of the costs. This is particularly true for the use of the same in developing countries. Hence, there is a need to provide systems and methods allowing to effect ELISPOT analysis under reduced costs, thus, enabling the production of inexpensive systems to be used in industrial and emerging as well as developing countries.

### Description of the present invention:

In a first aspect, the present invention relates to a device having multiple predetermined reaction areas wherein the device with the multiple predetermined reaction areas do not contain any membrane or structure present in or on the predetermined reaction areas, for use in an enzyme-linked immunospot (ELISPOT) assay.

In an embodiment, the present invention relates to a microtiter plate having multiple wells wherein the wells of the microtiter plate do not contain any membrane or structure fitted into the well, for use in an ELISPOT assay.

As used herein, the phrase "do not contain any membrane or other structure in or on the predetermined reaction area" or "do not contain any membrane or other structure fitted into the well" refers to the presence of any additional element introduced into or onto and at least temporally fixed in or on the reaction areas, like in the well, like a membrane, to improve binding and/or coating of the capture molecule.

The term "predetermined reaction area(s)" refer to specific area(s) in or on the device where the ELISPOT assay takes place.

That is, in contrast to the microtiter plate available in the art, the device with multiple predetermined reaction areas, like the microtiter plate having multiple wells are plain devices, like plain microtiter plates, e.g. are made of plastics and, in addition, are not pre-treated any further in so far that additional membrane elements or other structures for increasing the surface and/or for improving binding of capture antibodies or other affinity molecules are present.

The device, like the microtiter plate, according to the present invention allows to conduct ELISPOT assays where the number and quality of the spots is at least equal to the number and quality of spots using conventional ELISPOT plates including membranes. However, the costs are lower and, in addition, less steps are required as well as the steps are shortened in time.

This is in contrast to the general view of the art identifying that a crucial parameter include the use of membrane plates, in particular, PVDF-membrane plates, enabling efficient binding of high amounts of capture antibody. The present inventors recognized that in order to reduce the costs of an ELISPOT assay, conventional devices including conventional microtiter plates useful for normal cell culture can be used without any detrimental effect on the sensitivity and specificity of the assay.

That is, in contrast to the prior art, the device according to the present invention, like the microtiter plate to be used in the ELISPOT assay according to the present invention is e.g. a standard, typically, aseptical microtiter plate conventionally available. The device, like the plate, may be in a two part form. That is, the device like the microtiter plate may be composed of a baseplate, optionally having indentations for the reaction areas, and a frame forming the reacting areas or surrounding the reaction areas. The frame may form the multiple wells of the multiple well plate accordingly.

Thus, complex steps of coating of the microtiter plates containing a PVDF-membrane can be avoided. The PVDF-membrane requires pre-treatment of ethanol prior to coating, however, ethanol pre-treatment is connected with further errors beside the cost intensive use of the PVDF-membrane and the plates accordingly. That is, the ethanol pre-treatment can reduce the performance of the assay.

In contrast, when using the device of the present invention, e.g. in form of microtiter plates according to the present invention, this step can be omitted. According to the present invention, conventional microtiter plates or other conventional devices can be used.

Moreover, the PVDF membrane requires drying steps after stopping incubation and before reading out the results by elispot-reader or other devices.

In an embodiment of the present invention, the microtiter plate is a microtiter plate wherein at least the bottom of the inner surface of the well is coated with a capture molecule, like an antibody. Said capture antibody may be a polyclonal or monoclonal antibody. The antibody is typically specific for the secreted analyte to be determined.

In a further embodiment, the capture molecule, like the capture antibody is coated at least in the predetermined reaction areas of the device according to the present invention.

Typically, the capture antibody is an antibody capturing a cytokine secreted from the cells to be analysed. For example, a capture antibody is a polyclonal or monoclonal antibody directed against IL-2, IL-4, IL-17A, IL-22, IFN-gamma or TNF-alpha. Of course, the capture antibody itself is selected in a way to avoid any unspecific activation of the cells to be analysed.

In a preferred embodiment, the antibody is a monoclonal antibody binding specifically a cytokine, in particular, a cytokine selected from IL-2, IL-4, IL-17A, IL-22, IFN-gamma or TNF-alpha.

In a preferred embodiment, the capturing molecule is an aptamer or any other molecule having an affinity for the ligand to be tested, in particular, a cytokine selected from IL-2, IL-4, IL-17A, IL-22, IFN-gamma or TNF-alpha.

In a further embodiment, the device according to the present invention, like the microtiter plate having multiple wells, for use in an ELISPOT assay according to the present invention is a device, like a plate, made of plastic, in particular, polypropylene, polyethylene, polystyrene or mixtures thereof.

That is, the microtiter plate is a microtiter plate available for cell culture in general. The present inventors recognized that these non-modified cell culture microtiter plates, e.g. not containing a membrane or other structure fitted therein, allow determination of the cell product of interest with similar specificity and sensitivity as the ELISA test using cost intensive microtiter plates with PVDF-membrane.

The microtiter plate having multiple wells for use in the ELISPOT assay according to the present invention may be a microtiter plate wherein the bottom of the well is flat, U-shaped, V-shaped, F-shaped or C-shaped, or any other shape suitable for reading the spots. Depending on the cells and culture conditions, the skilled person will select the suitable shape of the bottom accordingly.

In an embodiment of the present invention, the microtiter plate having multiple wells for use in an ELISPOT assay according to the present invention is a microtiter plate wherein the multiple wells can be inserted or removed individually or sectionally of e.g. 8 wells of a row, from a frame of the microtiter plate. In the art, various embodiments of microtiter plates wherein the wells can be removed or inserted individually or sectionally from a frame of the microtiter plate are described.

In a further aspect, the present invention relates to an ELISPOT assay based method for determining compounds secreted from cultured cells. The method includes the culturing of these cells and said culturing is conducted in or on a device, like a microtiter plate as defined herein.

As used herein, the terms "including" or "include" or "comprising" or "comprise" as well as "contains" include the embodiment of consist or consisting of.

The method according to the present invention is particularly useful for detecting cytokines secreted from the cultured cells, typically cells activated with a specific antigen. For example, in case of ELISPOT for diagnosing mycobacterial infection like *Mycobacterium tuberculosis* infection, the antigen is derived from the mycobacterium like the mycobacterial tuberculosis, accordingly.

The method according to the present invention may comprise further the step of coating the device having predetermined reaction areas, like microtiter plate having multiple wells, with a capture molecule, like capture antibody, in particular, a capture antibody binding specifically a cytokine, before the step of culturing the cells. As identified above, the capture antibody is a polyclonal or monoclonal antibody, like polyclonal or monoclonal antibody binding specifically cytokines.

In addition, the ELISPOT assay based on the present invention may comprise further step of washing the wells for removing the cells, the cell debris and the culture medium.

Moreover, the method may include further steps of visualizing the bound secreted analytes bound to the captured antibody using e.g. a second labelled antibody binding specifically to the captured compound and, further means for visualizing the secreted analyte accordingly, as known in the art.

The ELISPOT based method according to the present invention is particularly for determining cytokine secreted analyte, in particular, IFN-gamma, IL-2, IL-4, IL-17A, IL-22, TNF-alpha.

As used herein, the terms "secreted analyte" and "secreted product of interest" are used interchangeably.

Moreover, the present invention relates to a test kit for use in an ELISPOT assay comprising a device, like a microtiter plate as defined herein, and, optionally, means for a visualizing compounds created from the cultured cells.

In addition, the test kit may include activating components, like antigen, for allowing antigen specific activation of immune cells. For example, the antigen is an antigen derived from mycobacterium including *Mycobacterium tuberculosis.* In particular, the antigen may be selected from AlaDH, ESAT6, CFP-10.

Moreover, the test kit according to the present invention may further comprise other components to be used in an ELISPOT assay method. These further components include assay buffer, washing buffer, labelled antibodies binding specifically to the compound to be determined, in particular, a capturing antibody, optionally, enzyme conjugate, substrate solution and stopping solution.

The test kit is particularly useful in a method as defined herein. Typically, the test kit includes microtiter plates of 96-well or 384-well. The microtiter plates are provided aseptically. Further, the test kit may include instructions to conduct the

ELISPOT assay accordingly.

The present invention will be described further by way of examples without limiting the same.

### Examples

### Comparison of microtiter plates having a membrane and a device according to the present invention.

Following materials and reagents are necessary for ELISPOT preparation with PVDF membrane containing micro titer plates and LUMITRAC#600 plates:

### Processing aids

| **Name** | **Supplier** | **Cat.-No.** |
|---|---|---|
| 25 mL sterile plastic pipet | Greiner | 760180 |
| 10 mL sterile plastic pipet | Greiner | 606180 |
| 5 mL sterile plastic pipet | Greiner | 607180 |
| 1,5 mL Eppendorf tubes | Greiner bio-one | 616201 |
| 15 mL Falcon tubes | Greiner | 188271 |
| 50 mL Falcon tubes | Greiner | 227261 |
| Plastic measuring cylinder | Omnilab | - |
| Plastic beaker | Omnilab | - |
| 15 mL Falcon tubes | Greiner | 188271 |
| 50 mL Falcon tubes | Greiner | 227261 |
| Schott bottles, 100 mL, 200 mL, 500 mL, 1 L, 2 L and 5 L | - | - |
| Leucosep tubes | Oxford Immunotec | LTK.615 |

### Devices

| **Name** | **see** |
|---|---|
| Centrifuge suitable for 15 mL and 50 mL falcon tubes | Cell preparation |
| Automatic cell counting machine | Cell preparation |
| Clean bench for sterile conditions | Cell preparation and application |
| 37°C incubator with 5 % CO₂ | Cell incubation |
| Microtiter plate automatic washer (optionally) | Washing of wells |
| Multichannel pipette | Sample- or cell application and washing of wells |
| 1000 µL vario pipette | Pipetting |
| 200 µL vario pipette | Pipetting |
| 100 µL vario pipette | Pipetting |
| 10 µL vario pipette | Pipetting |
| USB microscope | Visualization / Documentation of spots |

### Chemicals

| **Name** | **Supplier** | **Cat**.-**No**. |
|---|---|---|
| NaCl, ACS, Iso. Reag. Ph Eur | Merck | 1.06404.5000 |
| KCI, ACS, Iso. Reag. Ph Eur | Merck | 1.04933.1000 |
| Na2HPO4 x 2H2O, Acs, Reag. Ph Eur | Merck | 1.06346.1000 |
| KH2PO4, ISO, for Analysis | Merck | 1.04873.0250 |
| RPMI 1640 | Gibco | 72400-021 |
| FBS | Gibco | 10270-106 |
| Pen/Strep | VWR | K952-100ML |
| Ethanol, denatured | Roth | K928.1 |
| TMB Substrate solution | Seramun | S-002-5TMB |
| FICOLL (Lymphoprep) | Axis-Shield | AXS-1114545 |
| T-Cell Xtrend® reagent (optionally) | Oxford Immunotec | TT.610 |
| FilocethPLUS (optionally) | GERBU | 1903.0050 |

### Proteins

| **Name** | **Supplier** | **Cat**.-**No**. | **see** |
|---|---|---|---|
| mouse anti-human IL-2, monoclonal antibody | LIONEX | LAB a-IL-2b.mp mAB IgG | Coating antibody |
| mouse anti-human IL-2, monoclonal antibody, biotinylated | LIONEX | LAB a-IL-2h1.mp-B mAB IgG | Detection antibody |
| Streptavidin-Horseradish Peroxidase (HRP) | MABTECH | 3 of product code 3440-2A | Conjugate |
| PHA (phythohemagglutinin) | Gibco | 10576 | Positive control |
| AlaDH (Rv2780) | LIONEX | LRP-0022.5 | TB antigen |
| ESAT6 (Rv3875) | LIONEX | LRP-0017.3 | TB antigen |

### Other materials and buffers

| **PVDF membrane plate** | | **LUMITRAC#600** | |
|---|---|---|---|
| **Material** | | | |
| **Description** | **Supplier / Cat**.-**No**. | **Description** | **Supplier / Cat**.-**No**. |
| MultiScreen-IP, 0.45, Durapore, 8-well strips, sterile | Merck or Millipore / M8IPS4510 | LUMITRAC#600, Microtiterplate, 96 well, PS, | Greiner Bio-One / 655074 |
| | | F-Bottom, Chimney well, High Binding, white, sterile | |

| **Buffers** | | | |
|---|---|---|---|
| **Description** | | **Description** | |
| Equilibration buffer: | | No equilibration necessary | |
| 35% ethanol | | | |
| Washing after eqilibration, stop solution: | | Stop solution: | |
| ddH₂O | | ddH₂O | |
| Wash buffer: | | Wash buffer: | |
| 1x PBS, pH 7.4 | | 1x PBS, pH 7.4 | |
| Blocking solution: | | Blocking solution: | |
| 1x RPMI 1640 + 10% FBS + 1x Pen/Strep | | 1x RPMI 1640 + 10% FBS + 1x Pen/Strep | |
| Dilution buffer: | | Dilution buffer: | |
| 1x PBS, pH 7.4 + 0,5% FBS | | 1x PBS, pH 7.4 + 0,5% FBS | |

In the following, the procedure of ELISPOT preparation protocol for PVDF micro titer plates and LUMITRAC#600 are described.

### ELISPOT methodology

### General proceedings:

### Coating of PVDF membranes:

The PVDF micro titer plates should be prepared under sterile conditions. The membrane must be pre-wetted by addition of 30 µL of 35 % ethanol per well for exact 1 minute (according to the manufacturer specifications by Mabtech). After this, the plate have to be washed 5 times with sterile water (ddH₂O), each 200 µL per well. The wells must be equilibrated with ethanol again, if the wells dry. For coating, the antibody must be diluted in PBS, pH 7.4 (10 µg/mL). 100 µL per well of the diluted coating antibody solution must be added and incubated overnight at 2 - 8 °C (minimum 12 hours, maximum 24 hours).

### Coating of LUMITRAC#600:

The LUMITRAC#600 micro titer plates should be prepared under sterile conditions. The membrane does not need a pre-wetting step. For coating, the antibody must be diluted in PBS, pH 7.4 (10 µg/mL). 100 µL per well of the diluted coating antibody solution must be added and incubated overnight at 2 - 8 °C (minimum 12 hours, maximum 24 hours).

### Information to PBMC preparation:

It is recommended to isolate PBMCs within <8 hours after venipuncture to ensure high cell activity. For pre-longed storage of whole blood samples it is recommended to store the samples in the dark during gently agitation and in the best case, pre-diluted with medium (e.g. RPMI 1640^{®}) before storage starts. Another possibility is the use of T-Cell Xtrend^{®} reagent (Oxford Immunotec) for PBMC preparation after 8 hour storage. Avoid the use of whole blood samples older than >8 hours after venipuncture or too long storage of pre-diluted blood samples (>16 hours), this will cause low spot formation and not evaluable results.
In ELISpot test, PBMCs can be used freshly or thawed from nitrogen atmosphere. Repeated freezing and thawing of PBMC samples should be avoided.

### Recommended PBMC preparation protocol:

For small blood volumes use for example Leucosep tubes (Oxford Immunotec) for PBMC separation, otherwise it is also recommended to use FICOLL as preparation solution. For this dilute the whole blood volume 1:1 in 0.9 % NaCl infusion solution and layer
1) 6 mL of diluted blood over 3 mL FICOLL in 15 mL tube or
2) 30 mL of diluted blood over 20 mL FICOLL in 50 mL tube.

The separation fluid should not be admixed with the blood.

Centrifuge at 800 RCF (g) at room temperature in swing out rotor with the brake off (deceleration 0) for density gradient centrifugation as follows: for blood samples stored <2 hours for 20 min; for blood samples stored >2 hours for 30 min. Isolate the PBMC layer using 1000 µL vario pipette and transfer to new 15 mL or 50 mL tube.

Wash transferred PBMCs with 10-fold more volume of 1x PBS, pH 7.4. For this centrifuge for 10 min at room temperature as follows:
1) in 15 mL tube at 250 RCF (g) or
2) in 50 mL tube at 400 RCF (g).

After removal from the centrifuge, decant the supernatant and release/suspend the pellet without dilution in medium, just do it by flicking the tube several times. Resuspending by using of pipettes should be avoided. This will damage the cells.

Then add 10-fold volume 1x PBS, pH 7.4 to repeat wash step. During this time take a sample of PBMC/PBS suspension and count the cells.

### Cell application for PVDF and LUMITRAC#600:

The day after, excess antibody must be removed by washing the plates 5 times with sterile PBS solution, pH 7.4 with 200 µL per well. The wells should be blocked by using 200 µL per well of medium (1x RPMI-1640 medium + 10% FCS + 1x Pen/Strep) and incubated for at least 30 minutes at room temperature (sterile conditions). After this, the medium should be removed and 100 µL per well of cell suspension (250000 cells per well), plus 100 µL per well control solutions are added. As positive control PHA is diluted to final concentration of 1,5% in medium, as negative control only medium without any stimulating agents is used. For specific cell stimulation, 100 µL per well of TB antigens AlaDH and ESAT6 are used (pre-diluted in medium for final concentration of 40 µg/mL (AlaDH) and 5 µg/mL (ESAT6)). After application of cells plus controls and antigens, the plate must be transferred in a 37°C humidified incubator with 5% CO₂ and incubated overnight for minimum 16 to maximum 20 hours.

### Development of spots for PVDF and LUMITRAC#600:

For the spot detection, no sterile conditions are necessary. Washing of the plate (5 times with PBS solution, pH 7.4, each 200 µL per well) will remove all cell debris and unbound material. Addition of 100 µL per well of the diluted detection antibody solution (1 µg/mL in dilution buffer) and incubation for 1 hour at room temperature, is the next step. The antibody solution must be removed and the wells are washed 5 times as described above. 100 µL per well of the diluted streptavidin solution are added (3000x in dilution buffer) and incubated for 1 hour at room temperature. The streptavidin solution must be removed again and the wells should be washed 5 times as described above. Additionally, 100 µL per well of substrate solution (TMB) are added and incubated at room temperature until distinct spots are visible (10 minutes in the dark). As a final step, the colored reaction must be stopped by washing 3 times with 200 µL per well of stop solution.

### Interpretation of results by using PVDF membranes:

Leave the plate to dry. For this, use a ventilated area or an oven at up to 37°C. Allow 1 hour drying time at 37°C or at least 16 hours at room temperature. During this time, the background decreases and the spots become sharper. Counting of the spots could be done by using an ELISpot reader or USB microscope. Storage of the plate is possible in the dark at room temperature.

### Interpretation of results by using LUMITRAC#600:

Counting of the spots could be done right after removing of stop solution by using an ELISpot reader or USB microscope. No drying of wells is necessary for spot analysis. Storage of the plate is possible in the dark at room temperature.

Following the instructions above, examples have been conducted with PBMC as described. The examples show results of a standard ELISPOT micro titer plate containing PVDF membrane (MultiScreen-IP 0.45 Durapore 8-well strips, sterile, Cat.-No. M8IPS4510, Merck / Millipore) and plates of the present invention (LUMITRAC#600, Microtiterplate, 96 well, PS, F-Bottom, Chimney well, High Binding, white, sterile, Cat.-No. 655074, Greiner Bio-One) are shown in table 1.

**Table 1**

| | PVDF membrane plate | | | | LUMITRAC#600 | | | |
|---|---|---|---|---|---|---|---|---|
| Controls | Positive (PHA) | Positive (PHA) | Negative (medium) | Negative (medium) | Positive (PHA) | Positive (PHA) | Negative (medium) | Negative (medium) |
| IL-17 Spot counts | 294 | 328 | 0 | 0 | 262 | 240 | 2 | 2 |
| IL-22 Spot counts | 252 | 242 | 1 | 0 | 250 | 263 | 0 | 0 |
| Antigens | AlaDH | AlaDH | ESAT6 | ESAT6 | AlaDH | AlaDH | ESAT6 | ESAT6 |
| IL-2 Spot counts | 157 | 158 | 106 | 95 | 103 | 125 | 86 | 70 |

The number of spots from 100 to 500 in positive controls is considered "comparable number". Similarly, for negative controls, the number of spots from 0 to 20 is considered as "comparable number".

In comparison to the PVDF membrane micro titer plates, the LUMITRAC #600 micro titer plates need less preparation steps before use and allow an easier and faster way for result analysis.

That is, to prepare the binding of capture antibody onto the PVDF membrane micro titer plate additional steps are required. The wells need to be equilibrated by using diluted ethanol solution for a specific incubation time. After this, the wells have to be washed several times with washing solution to remove all remaining ethanol, otherwise the remaining ethanol may interfere the PBMC vitality. These steps are crucial for the PVDF membrane containing microtiter plate system since deviations in ethanol dilution as well as incubation time could lead to reduced test performance and false negative results.

In contrast, the device according to the present invention, e.g. the microtiter plate LUMITRAC #600, does not need any ethanol equilibration in advance. The capture antibody solution can be pipetted directly into the wells and incubated for binding. No need of ethanol removing or extra washing steps are necessary, which will accelerate the test preparation time.

Furthermore, after addition of the stop solution, the PVDF membrane need to be dried for reading out the test results. The drying time may be 1 hour at 37°C, or 16 hours at room temperature. Hence, additional time-consuming steps need to be performed when using PVDF membrane containing micro titer plate.

The advantage of the device according to the present invention, e.g. using the micro titer plate LUMITRAC #600, is that no drying time is necessary. The results are readable directly after removing of stop solution, which allows for fast analysis of test results.

As shown in the table, the spot counts show similar test results between PVDF membrane containing micro titer plate and LUMITRAC #600. Thus, the sensitivity between the costly membrane based system and the low prize and easy to handle device according to the present invention is similar. The negative controls does not show background signal in LUMITRAC#600 as well as in PVDF membranes. So the results are similar.

## Claims

1. A use of a device having multiple predetermined reaction areas wherein the device with the multiple reaction areas do not contain any membrane or other structure fitted into the well, for use in an enzyme-linked immunospot (ELISPOT) assay.

2. A use according to claim 1 wherein the device is a microtiter plate having multiple wells wherein the wells of the microtiter plate do not contain any membrane or other structure fitted into the well, for use in an ELISPOT assay.

3. The use of a device, like a microtiter plate according to claim 1 or 2
wherein at least predetermined reaction area, like the bottom of the inner surface of the well, is coated with a capture molecule, like a capture antibody.

4. The use of a device, like a microtiter plate having multiple wells for use in an ELISPOT assay according to claim 3 wherein the capture antibody is an antibody, preferably, a monoclonal antibody, binding specifically a cytokine.

5. The use of a device, like a microtiter plate having multiple wells, for use in an ELISPOT assay according to claim 4 wherein the cytokine bound by the antibody is selected from IFN-gamma, IL-2, IL-4, IL-17, IL-22, TNF-alpha.

6. The use of a device, like a microtiter plate having multiple wells, for use in an ELISPOT assay according to any one of the preceding claims wherein the device, like the plate, is made of plastic, in particular, polypropylene, polyethylene, polystyrene or mixtures thereof.

7. The use of a microtiter plate having multiple wells for use in an ELISPOT assay according to any one of claims 2 to 6 wherein the bottom of the well is flat, U-shaped, V-shaped, F-shaped or C-shaped.

8. The use of a microtiter plate having multiple wells for use in an ELISPOT assay according to any one of claims 2 to 7 wherein the multiple wells can be inserted or removed individually or sectionally from a frame of the microtiter plate.

9. An ELISPOT assay based method for determining compounds secreted from cultured cells wherein the culturing of said cells is conducted in or on a device, like a microtiter plate, as defined in any one of claims 1 to 8.

10. The ELISPOT assay based method according to claim 9 for detecting cytokines secreted from the cultured cells.

11. The ELISPOT assay based method according to claim 9 or 10 further comprising the step of coating at least the predetermined areas of the device, like the microtiter plate having multiple wells, with a capture molecule, like a capture antibody, in particular, a capture antibody binding specifically a cytokine, before the step of culturing the cells.

12. The ELISPOT assay based method according to any one of claims 9 to 11 further comprising the step of visualizing compounds bound to the captured antibody, in particular, using a second labelled antibody binding specifically to the compound captured with the capture antibody.

13. The ELISPOT assay based method according to any one of claims 9 to 12 wherein the cytokine to be determined is selected from IFN-gamma, IL-2, IL-4, IL-17, IL-22, TNF-alpha.

14. Test kit for use in an ELISPOT assay comprising a device, like a microtiter plate, as defined in any one of claims 1 to 8, and, optionally, means for visualizing compounds secreted from cultured cells, and, optionally, further comprising assay buffer, washing buffer, labelled antibody, binding specifically to the compound to be determined, in particular, a capturing cytokine, and, optionally, enzyme conjugate, substrate solution and stopping solution.

15. Test kit according to claim 14 for use in a method according to any one of claims 9 to 13.
